(19) **Europäisches Patentamt / European Patent Office / Office européen des brevets**

(11) **EP 2 795 495 B1**

(12) # EUROPEAN PATENT SPECIFICATION

(45) Date of publication and mention of the grant of the patent:
**04.09.2019 Bulletin 2019/36**

(21) Application number: **12821306.3**

(22) Date of filing: **07.12.2012**

(51) Int Cl.:
*G16H 40/63* (2018.01)    *G16H 50/30* (2018.01)
*G16H 50/20* (2018.01)    *G16H 50/50* (2018.01)
*A61B 5/00* (2006.01)    *G06N 5/02* (2006.01)

(86) International application number:
**PCT/IB2012/057076**

(87) International publication number:
**WO 2013/093692 (27.06.2013 Gazette 2013/26)**

(54) **METHOD AND SYSTEM TO PREDICT PHYSIOLOGIC AND CLINICAL STATUS CHANGES**

VERFAHREN UND SYSTEM ZUR VORHERSAGE VON PHYSIOLOGISCHEN UND KLINISCHEN ZUSTANDSÄNDERUNGEN

PROCÉDÉ ET SYSTÈME DE PRÉVISION DE CHANGEMENTS D'ÉTAT CLINIQUE ET PHYSIOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2011 US 201161578512 P**
**17.02.2012 US 201261600140 P**

(43) Date of publication of application:
**29.10.2014 Bulletin 2014/44**

(73) Proprietor: **Koninklijke Philips N.V.**
**5656 AE Eindhoven (NL)**

(72) Inventors:
• **GROSS, Brian David**
**NL-5656AE Eindhoven (NL)**
• **ESHELMAN, Larry James**
**NL-5656 AE Eindhoven (NL)**
• **FLOWER, Abigail Acton**
**NL-5656AE Eindhoven (NL)**

• **CHIOFOLO, Caitlyn Marie**
**NL-5656AE Eindhoven (NL)**
• **LEE, Kwok Pun**
**NL-5655 AE Eindhoven (NL)**
• **CAO, Hanqing**
**NL-5656AE Eindhoven (NL)**
• **FRASSICA, Joseph**
**NL-5656 AE Eindhoven (NL)**
• **NIELSEN, Larry**
**NL-5656AE Eindhoven (NL)**
• **SAEED, Mohammed**
**NL-5656AE Eindhoven (NL)**

(74) Representative: **Steffen, Thomas**
**Philips Intellectual Property & Standards**
**High Tech Campus 5**
**5656 AE Eindhoven (NL)**

(56) References cited:
**WO-A1-2006/136972    WO-A1-2011/082421**
**US-A1- 2006 025 931    US-A1- 2008 188 733**
**US-A1- 2009 210 163**

## Description

The present application relates to clinical decision support. It finds particular application in conjunction with predicting physiological and clinical status changes and will be described with particular reference thereto. However, it is to be understood that it also finds application in other usage scenarios and is not necessarily limited to the aforementioned application.

When caring for patients, the sooner a clinician learns of deterioration of a patient, the sooner the clinician can take corrective action. This, in turn, reduces the likelihood of end organ damage, especially in the case of hemodynamic deterioration, and generally improves patient outcome. To detect deterioration typically requires a clinician to manually review physiological data for a plurality of physiological parameters, such as systolic blood pressure, and/or laboratory data. However, clinicians care for a large number of patients and the ratio of patients to clinicians is only expected to increase. Further, the frequency with which the physiological data is generated is high. As such, clinicians are often delayed in detecting deterioration.

To alleviate this, automatic monitoring of patients is becoming increasingly prevalent. However, a principal challenge with automatic monitoring is alert fatigue. Alert fatigue is the condition in which clinicians become desensitized to clinical alerts because of the high probability that alerts are not of actual clinical significance. A simple solution is to raise alert thresholds. However, this reduces sensitivity and increases the likelihood of failing to detect patient deterioration.

Another solution is to set an inhibition period after an alert issues, so similar alerts are not issued until a rearming condition is met. In such an approach, the rearming condition is crucial to reducing the alerts. The typical rearming condition is the passing of a predetermined amount of time from the alert triggering the inhibition period and then reevaluating the physiological data after this period has passed. This is based on the notion that any alert following the first alert is likely to be based on similar physiological data, and thus does not provide any additional information to the clinician. The clinician either is already planning to take action to treat the patient if he/she agrees with the alert or doubts the validity of the alert and in either case would find another alert unnecessary. Thus it is reasonable to inhibit further alerts.

One disadvantage of this alerting solution is that additional alerts are not raised if the condition of a patient worsens within the inhibition period. Another disadvantage is the predetermined amount of time is typically fixed. As such, the predetermined amount of time is not tailored to any specific patient. Further, the predetermined amount of time does not adapt to an individual's physiologic dynamics or interventions.

Other challenges with automatic monitoring stem from predictive models typically employed by automatic monitoring systems. Such predictive models are typically trained on large databases of population data, whereby decisions using such predictive models are based on the general features of a large population. Further, differences between individuals and the general training population are typically not taken in to account. Training in this way can result in unnecessary alerts and/or failure to generate alerts for certain patients with physiological norms different from those of the general training population.

Mitigating this, direct feedback from a clinician about the validity of an issued alert can be employed for learning. However, such an approach is not possible for systems that do not have the benefit of this direct-feedback learning. Further, if an alert is issued in response to predicted events hours in advance, immediate feedback from a clinician about the validity of the alert is meaningless.

WO 2006/136972 pertains to a patient monitoring system wherein a composite acuity score generator generates or updates respective score indicative of wellbeing of the patient and/or deterioration based on sensed physiological parameters and longer interval data. Different data indicated on the monitor and deterioration may cause an alert.

The present application provides new and improved methods and systems which overcome the above-referenced problems and others.

In accordance with one aspect, a clinical decision support system for monitoring one or more patients is provided. The system includes one or more processors programmed to receive patient data from the patients. For each patient, one or more monitoring rules are selected from a plurality of monitoring rules based on vital signs instability index value of said patient. A determination as to whether a patient is deteriorating is made using the selected monitoring rules for the patient. In response to determining the patient is deteriorating, an alert is generated.

A baseline vital signs instability index (VIX) is calculated for the patient from VIX values within a past predetermined amount of time; the baseline VIX indicates how the VIX has been behaving within the past predetermined amount of time; the baseline VIX is compared to the VIX threshold; and in response to the VIX exceeding the VIX threshold and the baseline VIX exceeding a predetermined fraction of the VIX threshold, deteriorating of the patient is determined.

In accordance with another aspect, a method for monitoring one or more patients is provided. The method includes receiving patient data for the patients. For each patient, one or more monitoring rules are selected from a plurality of monitoring rules based on vital signs instability index value of said patient. A determination as to whether a patient is deteriorating is made using the selected monitoring rules for each patient. In response to determining the patient is deteriorating, an alert is generated for that patient.

A baseline VIX is calculated for the patient from VIX values within a past predetermined amount of time; the baseline VIX indicates how the VIX has been behaving within the past predetermined amount of time; the baseline VIX is compared to the VIX threshold; and in response to the VIX exceeding the VIX threshold and the baseline VIX exceeding a predetermined fraction of the VIX threshold, deteriorating of the patient is determined.

In accordance with another aspect, a system for assessing the stability of a physiological condition of a patient is provided. The system includes one or more processors programmed to receive patient data for the patient. The patient data includes monitoring data and patient specific contextual data. The system conditions the raw data so they can be utilized in the predictive algorithm system. A vital signs instability index (VIX) for the physiological condition is calculated from the monitoring data using a predictive model. A VIX threshold of instability is determined based on available lab data. A determination as to whether the patient is unstable is made at least partially by comparing the VIX to the VIX threshold. In response to determining the patient is unstable, an alert is generated.

A baseline VIX is calculated for the patient from VIX values within a past predetermined amount of time; the baseline VIX is compared to the VIX threshold; and in response to the VIX exceeding the VIX threshold and the baseline VIX exceeding a predetermined fraction of the VIX threshold, deteriorating of the patient is determined.

One advantage resides in a stability index taking into account multiple physiological parameters.

Another advantage resides in reducing alerts.

Another advantage resides in focusing a clinician's attention on patients requiring extra vigilance.

Another advantage resides in processing low-latency data separately from high-latency data.

Another advantage resides in increased sensitivity to abnormal patient conditions.

Another advantage resides in adaptability to available data.

Another advantage resides in reducing the likelihood of outlying values triggering alerts.

Another advantage resides in adjusting to cases in which a patient has conditions that are not typical of the average population.

Still further advantages of the present invention will be appreciated to those of ordinary skill in the art upon reading and understand the following detailed description.

The invention may take form in various components and arrangements of components, and in various steps and arrangements of steps. The drawings are only for purposes of illustrating the preferred embodiments and are not to be construed as limiting the invention.

FIGURE 1 is a block diagram of an information technology (IT) infrastructure of a patient care environment.

FIGURE 2 is a block diagram of a clinical decision support system.
FIGURE 3 is a graphical representation of a vital signs index for hemodynamic stability and the corresponding inputs.
FIGURE 4 is a graphical representation of a baseline vital signs index and a vital signs index.
FIGURE 5 is a graphical representation of a vital signs index for hemodynamic stability and the corresponding inputs.
FIGURE 6 is a flow chart of a clinical decision support system.
FIGURE 7 is a block diagram of a method for monitoring one or more patients.

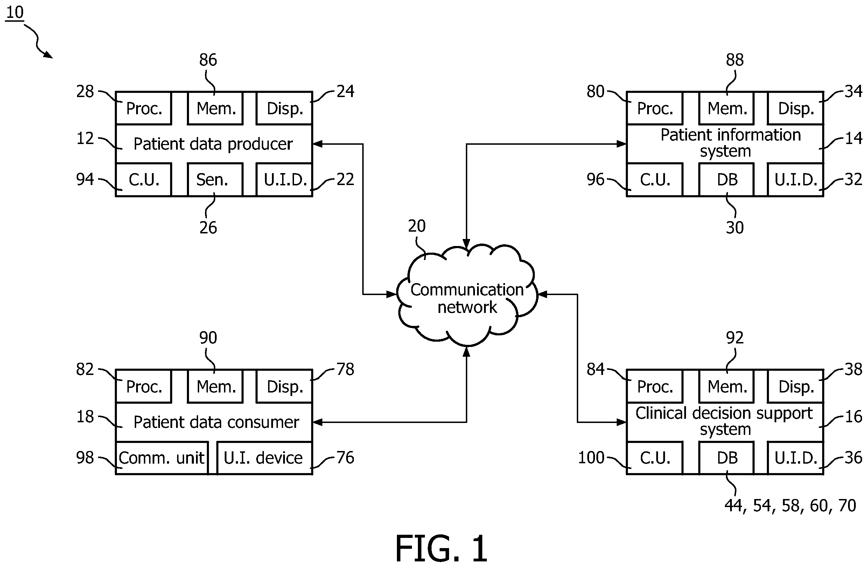

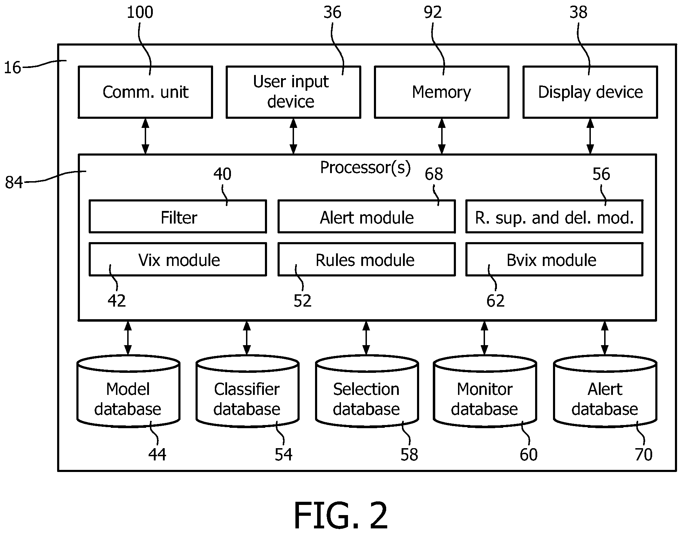

With reference to FIGURES 1 and 2, an information technology (IT) infrastructure 10 of a patient monitored environment, such as an intensive care unit (ICU), caring for one or more patients is provided. The IT infrastructure 10 includes one or more patient data producers 12, optionally a patient information system 14, a clinical decision support system (CDSS) 16, one or more patient data consumers 18, and the like. Suitably, the components of the IT infrastructure 10 are interconnected via a communication network 20, such as the Internet, a local area network, a wide area network, a wireless network, a virtual private network, or the like.

The patient data producers 12 generate patient data for corresponding patients cared for in the patient monitored environment. The patient data for a patient includes one or more of physiological data, admission, discharge and transfer (ADT) data, laboratory data, clinical data, outcome data, and alert data. Physiological data includes one or more of physiological parameter waveforms, measurements of physiological parameters typically generated at a predetermined sample rate, such as 1 second or upon a change in the parameter, observed physiological parameters, and calculated physiological parameters. Examples of such physiological parameters include systolic blood pressure (SBP), heart rate (HR), SpO2, and so on. ADT data includes values for demographic data, such as age, nationality, and so on, and care preferences, such as do not resuscitate (DNR), comfort measures only (CMO), allow natural death (AND), reason for admission to the hospital or care unit, and so on. ADT data is typically generated when a patient is admitted to and/or discharged from a medical institution and/or transferred between patient care environments, such as the ICU and/or general ward, of a medical institution. Laboratory data includes laboratory test results and is typically generated sporadically upon the happening of an event, such as a clinician ordering a lab test. Clinical data includes data indicating actions taken to improve the health of the patient and other observations such as level of consciousness, intervention measures, dialysis, medications administered, the patient social and prior medical history, history of current illness, other social or risk factors the patient possesses, such as genomics, and so on. Outcome data includes data indicating the outcome of a patient's medical treatments and/or stay in the medical institution, such as whether the patient's condition worsened or improved, whether the patient died, and so on. Typically, the outcome data is generated during the patient's stay at a medical institution after medical interventions are taken. Alert data is data indicating an alert, such as deterioration of a patient, and is typically generated in response to detection of deterioration.

The patient data can be generated manually and/or automatically, typically depending upon the type of patient data. As to the former, user input **22** can be employed. Optionally, the patient data producers **12** include display devices **24** providing users with a user interface within which to manually enter the patient data and/or for displaying patient data to clinicians. As to the latter, sensors **26**, such as SpO2 sensors, measuring, for example, physiological parameters and/or lab results and/or processors **28** monitoring and/or processing data, such as patient data, can be employed. Examples of patient data producers include, but are not limited to, physiologic monitors, mobile communications devices, laboratory systems, point of care systems, clinical information systems, and so on.

The patient information system **14** stores patient data from the IT infrastructure **10**, such as from the patient data producers **12** and/or the CDSS **16**, in one or more databases **30** of the IT infrastructure **10**. For example, the patient information system **14** can store SBP for a patient from one of the patient data producers **12**. The patient information system **14** is configurable to store patient data from user input devices **32** in the databases **30** and/or to allow stored patient data to be viewed on display devices **34**. The display devices **34** can also be used to facilitate receipt of data from the user input devices **32**. Suitably, the patient information system **14** manually stores patient data for a predetermined amount of time, such as a year, to allow other components of the IT infrastructures **10**, such as the CDSS **16**, to access historical patient data. Examples of patient information systems include, but are not limited to, electronic medical record systems, departmental systems, and the like.

The CDSS **16** receives patient data for the patients from the IT infrastructure **10**, such as from the patient data producers **12** and/or the patient information system **14**. The CDSS can be alternatively configured so that patient data is also received from other sources, such as user input devices **36**, optionally with display devices **38** providing users a user interface within which to enter the patient data, and/or sources, such as databases, external to the IT infrastructure **10**. The patient data includes, for example, current patient data (e.g., current measurements of physiological parameters) and/or historical patient data (e.g., prior measurements of physiological parameters). Using the received patient data, the CDSS **16** monitors the wellbeing of the patients. Monitoring includes, for example, generating alerts when the condition of a patient appears to be deteriorating, reports summarizing the state of the patients, vital instability index (VIX) values, and so on.

CDSS 16 optionally also includes a filter 40. At least some of the received patient data passes through the filter **40** that conditions patient data into a standardized format and/or filters patient data that is not suited to monitoring the wellbeing of the patients. Advantageously, conditioning patient data allows the CDSS **16** to be utilized in a variety of hosts and consume data from a variety of hosts in the native format. The filtering can include one or more of comparing the patient data to predetermined ranges of normalcy, ensuring the patient data meets time criteria for usability, and cross checking the patient data. For example, physiological data is typically filtered to remove measurements that aren't within predetermined boundaries, such as boundaries indicating possible values, and/or are otherwise highly unlikely. As another example, ADT data is typically filtered to remove ADT for patients that do not belong to a targeted demographic (e.g., age, ethnicity, etc.). For example, ADT data for a patient which has not reached a predetermined age, such as the age of majority (e.g., generally 18 in the United States), and/or which exceeds a predetermined age, such as an improbable or unlikely age. As yet another example, laboratory results are typically filtered to remove results that aren't within predetermined boundaries, such as boundaries indicating possible values, and/or which exceed a predetermined age, such as 24 hours. Advantageously, this removes data which is stale and/or likely to be outlying thereby reducing the likelihood of false alarms.

A VIX module **42** of the CDSS **16** calculates VIX values from the received patient data (as filtered, where relevant). VIX typically combines low-latency data, such as current physiological data, for a plurality of physiological parameters and, optionally, static data, such as demographic data, into a single measure reflective of stability of a physiological condition of a patient, such as the patient's hemodynamic status, pulmonary stability, nutritional stability, and so on. The CDSS **16** can be configured such that the VIX values for the patients are displayed on the display devices **38**. The VIX values can be calculated continuously and/or upon the happening of an event, such as a timer event, a user input event, the availability of new data, and so on. For example, a clinician can manually trigger calculation of a VIX for a patient so as to determine the hemodynamic stability of the patient. The CDSS **16** can further be configured such that the VIX values are saved for historical analysis, typically in the patient information system **14**.

A VIX value for stability of a physiological condition is calculated by providing values for predictive variables to a selected VIX model that generates the VIX value based on the predictive variables. The predictive variables are one or more of physiological parameters, features extracted from the static data, such as ethnicity, and the like relevant to determining the stability of the physiological condition. Suitably, the VIX model is selected from a plurality of VIX models in a VIX model database **44** based on the physiological condition and/or the availability of data. For example, a first VIX model is selected for stability of a first physiological condition and a second VIX model is selected for stability of a second physiological condition. As another example, a first VIX model is selected when measurements for noninvasive SBP and HR are available and a second VIX model is selected when invasive SBP and HR are available. Further, the VIX values produced by the models typically range between 0 and 1, where the closer the value is to 1, the more likely the patient is to be unstable. The VIX models can be developed using any predictive model methodology, such as logistic regression, multinomial logistic regression, linear regression and support vector machine learning.

The generic VIX models include for instance a logistic regression model for hemodynamic instability with the form of:

$$VIX = \frac{1}{1 + e^{-z}},$$

where

$$z = \gamma + \beta_1 * SBP + \beta_2 * SI + \cdots$$

Specific VIX models are derived from different patient sub-populations (cardiogenic shock, hemoragic shock, septic shock and so on), and based on different source parameters (invasive SBP, non-invasic SBP, and the like). The model takes into account SBP and SI, which are highly significant predictive variables in determining hemodynamic stability, and returns a VIX between zero and one. The higher the VIX, the less stable the patient. In some instances, $\beta_1$, the coefficient for SBP, is negative. As SBP gets lower, VIX tends to increase, reflecting that the patient is approaching a less stable state. Further, $\beta_2$, the coefficient for SI, is positive. As SI gets higher, VIX also tends to increase, again reflecting a decrease in stability. An approach for determining the coefficients is discussed hereafter.

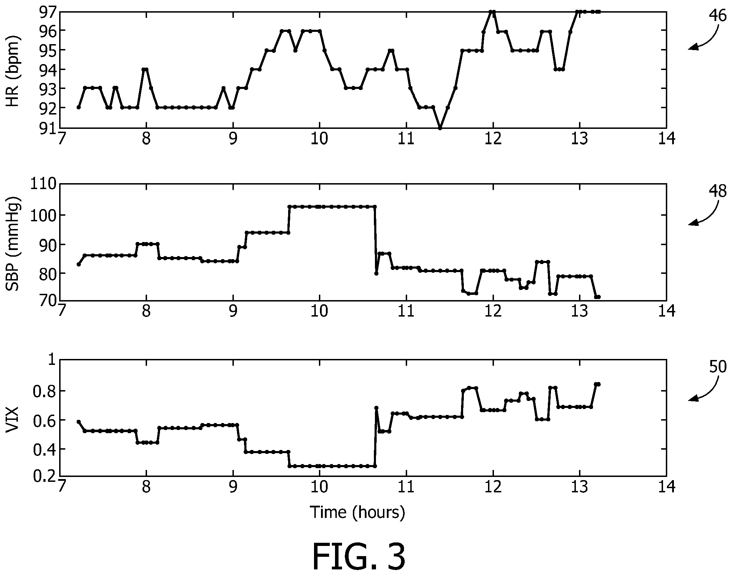

With reference to FIGURE 3, a typical VIX output for hemodynamic stability and the corresponding inputs as a function of time are illustrated. The inputs were drawn from real patient data. A first panel **46** and a second panel **48** of the plot show the HR and SBP, respectively, of a patient. As noted above, HR and SBP are highly significant predictive variables in determining hemodynamic stability. In a third panel **50**, the calculated VIX values are displayed. One can see that, by hour 11.75, the patient's VIX is rather high (nearly 0.8), indicating that the patient is not hemodynamically stable. Although not illustrated, at hour 14, the patient was administered a vasopressor, indicating that the hemodynamic instability was recognized by the clinician.

Referring back to FIGURES 1 and 2, the VIX module **42** can be further configured to generate and/or update the VIX models from historical patient data. The historical patient data includes records for a plurality of patients, where each records includes values for a plurality of variables, including the predictive variables, and corresponding outcome data indicating whether the patient was stable for the physiological conditions of the predictive models being updated and/or generated. Using the records, specifically the associations between the variables and the outcomes, and a predictive model methodology, such as logistic regression, multinomial logistic regression, linear regression and support vector machine learning, the predictive variables and/or one or more rules for predicting outcome are identified. For example, multivariable logistic regression can be employed to identify the predictive variables and the coefficients of the above described logistic regression model. When the VIX module **42** is employed to update the VIX models, the VIX models are typically updated in response to an event, such as a periodic timer event, a user input event, the availability of new historical patient data, and so on.

A rules module **52** of the CDSS **16**, determines VIX thresholds indicating instability for the patients. A VIX threshold for a patient is determined based upon the corresponding physiological condition and, optionally, contextual data, such as laboratory data and/or demographic data. Contextual data is data describing one or more of where a patient is in the care process, the patient's problem list, interventions, demographics, laboratory tests, and the like. Contextual data is not directly relevant to the VIX, but provides an indication as to where the threshold should be set for a particular patient. For example, a patient with a creatinine value of 0.9 mg/dL may be considered stable with a VIX value of .5, whereas a patient with a creatinine of 3.2 mg/dL may not. When no contextual data regarding a patient is available, a generic threshold is employed for the patient. Otherwise, a threshold based on the provided contextual data is typically employed. For example, if contextual data, such as blood lab results that indicate a low hematocrit or albumin level, are generated, the VIX threshold can be adjusted to a lower value.

The VIX thresholds are suitably determined from rules of one or more VIX classifiers of a VIX classifier database **54** which discriminate between stability and instability given values for a plurality of variables, including VIX and, optionally, one or more variables of contextual data, such as laboratory tests. The rules module **52** can optionally include a VIX classifier for each possible set of input variables. For example, the rules module **52** can include a VIX classifier for an input comprised of only a VIX value and a VIX classifier for an input comprised of a VIX value and a first contextual value, such as a lab result for a particular lab test. The VIX classifiers can be generated and/or updated using any machine learning methodology, such as decision tree algorithms. Advantageously, any rule set for discriminating between stable and unstable patients using decision tree analysis will have a background rule, indicating a threshold for VIX in the absence of any contextual data. For example, if VIX is greater than 0.6, the patient is unstable. In addition to this background rule, the rule set includes rules incorporating contextual data, such as laboratory, and increasing or decreasing the VIX threshold depending upon the context. For example, if VIX is greater than 0.33 and creatine is greater than 1.6, the patient is unstable.

The rules module **52** may also generate and/or update the VIX classifiers from historical patient data. The historical patient data includes records for a plurality of patients, where each record includes values for input variables, including VIX and, optionally, variables of contextual data, such as laboratory tests, having a bearing on the monitoring sensitivity for the patient, and outcome data indicating whether the patient was unstable. Using the records, specifically the associations between the input variables and the outcomes, and a machine learning algorithm, such as a decision tree algorithm, one or more rules for determining the outcome are determined. When the rules module **52** is employed to update the classifiers, the VIX classifiers are typically updated in response to an event, such as a periodic timer event, a user input event, the availability of new historical patient data, and so on.

A rule supervisor and selector module **56** determines a set of one or more monitoring rules and/or one or more VIX models to employ for each patient to be monitored. A monitoring rule takes as input values for one or more variables, such as a physiological parameter, and provides an indication as to whether a patient is deteriorating. To determine a set of one or more monitoring rules and/or one or more VIX models, one or more selection rules of a selection rules database **58** are employed. The selection rules select one or more monitoring rules from a plurality of monitoring rules in a rules monitoring database **60** and/or select one or more VIX models from the VIX database **44**. The monitoring rules are suitably generated manually by, for example, a clinical expert and/or automatically using a machine learning algorithm.

The selection rules can be based upon one or more of available patient data, patient context, and/or the source of the patient data. Typically, however, the selection rules are based upon contextual data, such as laboratory data and/or demographic data. Where contextual data is available, the selection rules determine a set of monitoring rules and/or VIX models tailored to the available contextual data. Where contextual data is unavailable, the selection rules return a generic set of monitoring rules and/or a generic VIX model selection. In this way, the rule supervisor and selector module **56** is adaptive to available patient data. While selection rules based on contextual data are typical, other selection schemes are contemplated. For example, it is contemplated that the rule supervisor and module **56** simply returns a set of monitoring rules for every patient regardless of the availability of contextual data. Similar to the monitoring rules, the selection rules are suitably generated manually by, for example, a clinical expert and/or automatically using a machine learning algorithm.

The monitoring rules may be based on VIX values. However, one challenge with employing VIX values is that a high VIX value can be the result of outlying physiological data due to, for example, a misplaced monitoring lead or a patient's sudden change in arm position, or movement from a prone to a sitting or standing position. Especially where automatic patient monitoring is employed, such data can lead to false alerts. To reduce the likelihood of these false alerts, a baseline VIX (bVIX) indicating how the VIX has been behaving is calculated by a bVIX module **62** of the CDSS **16**. The bVIX module **62** calculates bVIX values from historical patient data. Many methods can be used to estimate the trend in a series of VIX values. Some are more sophisticated than others. In one example, the bVIX value is the maximum VIX value or the 90 percentile value within the past predetermined amount of time, such as three hours.

When a bVIX is employed, an alert exceeding a trigger threshold is indicated only if a bVIX indicates a rising trend in VIX. Then the current high VIX value is more likely to reflect the true state of the physiological condition. Otherwise a false alert is more likely. In one example, a rising trend is detected when the current VIX value is more than a predetermined threshold and bVIX is at least some fraction of the threshold (e.g., 3/4 or 2/3). This means the current VIX is high enough to cause alarm and an earlier VIX value is already quite high. Then and only then is an alert raised. If bVIX is too low, then the current VIX, though high, is more likely to be an aberration and no alarm is raised. This specific implementation has the advantage of being simple and efficient and has proved to be effective in reducing alerts due to data outliers.

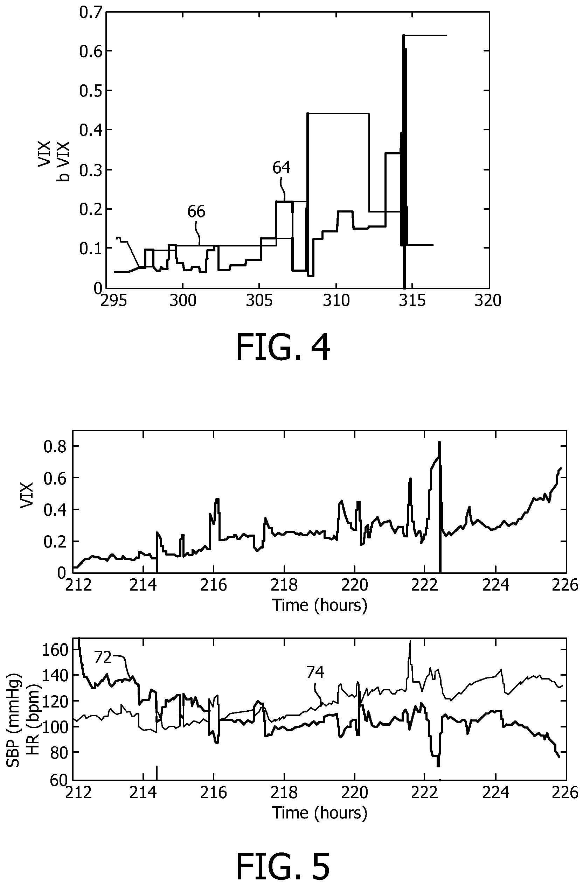

With reference to FIGURE 4, an example of VIX values **64** and the corresponding bVIX values **66** calculated for a patient during a portion of their stay in the patient care environment as a function of time since admission is provided. With reference to around hour 308, the patient's VIX value reaches a high enough value to trigger an alert. However, because the bVIX value at this point in time (~0.22) is too low, no alarm is triggered as a result of this spike in VIX. In this way, the implementation of bVIX has made it impossible to trigger an alert due to this quite possibly aberrational data. Later in the patient's stay, around hour 314.5, the patient's VIX again becomes high enough to cross a threshold for instability. This time, however, the bVIX is also high enough (~0.4) to allow an alarm to be triggered. Essentially, bVIX is sensitive to trends in data; a high VIX resulting from a gradual increase in VIX values is far more likely a result of actual important physiological changes than is a VIX that suddenly increases.

Referring back to FIGURES 1 and 2, an alert supervisor module **68** monitors the patient data for the patients and generates alerts when deterioration is detected. To determine when a patient is deteriorating, the alert supervisor module **68** employs the set of monitoring rules for the patient determined by the rule supervisor and selector module **56**. As noted above, the monitoring rules take as input values for one or more variables, such as physiological parameters, typically received from the IT infrastructure **10**. Further, the input variables can include VIX. The VIX values are received from, for example, the VIX module **42** and generated, for example, according to the VIX model selections made by the rule supervisor and selector module **56**. Further, the thresholds for the monitoring rules for the VIX values are received from the rules module **52**.

When it is determined that a patient is deteriorating, an alert to that affect is generated. The alert is suitably generated and addressed to a clinician according to one or more rules in an alert rules database **70**. The rules can take into account one or more of hospital policy, clinician worklists, on-call status of clinicians, clinician preferences, and so on. For example, suppose hospital policy specifies that in response to an alert of a particular type, alert the on-call physician overseeing the physician. Further, suppose the on-call physician wishes to be contacted in a particular way, such as text message. The rules can be employed to generate and send an alert meeting these requirements. Alert escalation is also contemplated. Alert escalation is the idea of escalating an alert by resending it to the same or a different clinician after conditions, such as the passing of a predetermined amount of time without receiving an acknowledgment, are met.

In addition to sending the alert, alerts for the same deterioration are disarmed (i.e., prohibited from being triggered) until a rearming condition is met. The rearming conditions can include, for example, the passage of a predetermined amount of time. Further, the rearming conditions can, for example, be determined by an adaptive rearming method. The adaptive method presupposes familiarity with the typical intervention measures taken by clinicians in response to deterioration of the physiological condition corresponding to the disarmed alert and the availability of clinical data indicating interventions taken by clinicians. Further, the adaptive method presupposes an index or parameter that reflects a patient's stability with regard to the physiological condition. For example, when the physiological condition is hemodynamic stability, the index or parameter can be VIX and typical intervention measures include the administration of fluids, vasopressors, or packed red blood cells.

According to the adaptive method, if an alert for deterioration of a physiological condition is issued, alerts for the same deterioration are prohibited from being issued for a predetermined amount of time, such as three hours. The predetermined amount of time corresponds to the lead time of the prediction that deterioration will occur and typically varies depending on the physiological condition. After the predetermined amount of time has passed, a determination is made as to whether intervention measures have been taken to address the deterioration based on clinical data typically received from the IT infrastructure **10** and knowledge of typical intervention measures for the physiological condition.

If no intervention has been or is being administered, alerts for the same deterioration are disarmed unless and/or until the index or parameter has worsened by a threshold amount compared to the index or parameter value at the time of the initial alert. The threshold amount can be fixed or variable, such as half of the distance to a previous index or parameter value. By rearming in this way, the lack of intervention by a clinician after a significant time has passed is interpreted as an indication that the patient's condition at the time of the first alert is acceptable for that particular patient. Therefore, even though the index or parameter is abnormal, or unstable, by population standards, the adaptive method learns that it is normal. If intervention is being administered, this is recognized as acknowledgement by clinicians and further alerts are unnecessary. After the intervention has ended, alerts for deterioration of the physiological condition are rearmed after a predetermined amount of time passes.

With reference to FIGURE 5, a plot of the VIX for a patient and the corresponding inputs, SBP **72** and HR **74**, over the course of several hours is illustrated. An alert is generated at hour 214.5 as the VIX value of the patient crosses the alert threshold. During the course of the three hours that follows the first alert, no intervention is taken. This is interpreted to mean that the patient's dynamics at the time of the first alert are acceptable for that particular patient. Therefore, after three hours have passed, no alert is generated because the patient's VIX is not much higher than it was at the time of the first alert. By the time hour 222.5 is reached, however, VIX has significantly increased and, therefore another alert is issued for the patient. It should be noted that at hour 226, the clinician administered vasopressor indicating that, indeed, the patient experienced a clinically notable incident of hemodynamic instability.

Referring back to FIGURES 1 and 2, the patient data consumers **18** consume patient data from the IT infrastructure **10**, such as from the patient data producers **12**, the CDSS **16**, the patient information system **14**, and so on, for the patients. For example, the patient data consumers **18** can receive VIX values from the CDSS **16**. As another example, the patient data consumers **18** can receive respiration rate and heart rate from the patient data producers **12**. As yet another example, the patient data consumers **18** can receive alerts from the CDSS **16**. Optionally, the patient data consumers **18** also receive patient data from user input devices **76**, optionally with display devices **78** providing users a user interface within which to enter the patient data. Examples of patient data consumers include, but are not limited to, patient monitors, spot check patient monitors, mobile communications devices, patient information systems, clinical decision support systems, and so on.

Consumption can include processing the received patient data to generate additional patient data and/or consolidating the patient data into reports. A report is a computer file in a format, such as PDF, DOCX, DOC, and so on. Optionally, newly generated patient data and/or newly generated reports are saved in the IT infrastructure **10**, such as in the patient information system **14**. Further, optionally, newly generated reports are electronically messaged to clinicians using, for example, email and/or printed using, for example, a laser printer, an inkjet printer, and so on. Consumption can also include displaying the received patient data, such as alerts or VIX values, for at least one patient on a user interface presented to clinicians via the display devices **78**. The user interface is typically continuously updated as patient data is received. Advantageously, this allows clinicians to monitor patients in near real time.

When displaying patient data and/or generating a report, the report and/or display suitably includes at least patient name and VIX values for at least one patient. Where the received patient data includes patient data for a plurality of patients, the received patient data is suitably formatted in a table structure with a plurality of rows corresponding to the patients. The rows can optionally be sorted and/or can be sorted by severity of VIX. For example, a clinician can employ the user input devices 76 to sort a table of patient data based on VIX. Further, clinicians can optionally selectively view the details of a VIX. For example, a clinician can employ the user input devices **76** to select a VIX for a patient and view the variables and respective values that yielded the VIX, optionally ranked based on contribution. Even more, the patient data can optionally be grouped based on similar VIXs. Groups include, for example, one or more of very low risk, low risk, moderate risk, high risk, and so on.

A VIX can be represented as one or more of textual values (e.g., scores, probabilities, and so on), icons (e.g., one or more of shape, color, background, and so on based on severity), a combination of the foregoing, and so on in a user interface and/or a report. For example, a VIX can be represented as a circle having a background color dependent upon severity, such as red for high risk, yellow for medium risk, and green for low risk. An icon can further includes a textual value overlaid thereon, optionally depending upon severity. For example, an icon can include a probability overlaid thereon when the severity is medium. A VIX can also be presented to the use as a discrete message (e.g., alert, text page, email, SMS, and so on) or as a parameter based on the absolute probability that the patient will have an insatbility event in a preconfigured or continuous time horizons, or as a normalized scale of overall prediction of instability based on VIX and other CDSS algorithms running for the patient.

The components of the IT infrastructure **10** suitably include processors **28, 80, 82, 84** executing computer executable instructions embodying the foregoing functionality, where the computer executable instructions are stored on memories **86, 88, 90, 92** associated with the processors **28, 80, 82, 84.** The processor(s) **84** of the CDSS **16**, for example, execute computer instructions on the one or more memories **92** of the CDSS **16** embodying the functionality of one or more of the filter **40**, the VIX module **42**, alert module **68**, the rules module **52**, the rule supervisor and selector module **56** and the bVIX module **62**. It is, however, contemplated that at least some of the foregoing functionality can be implemented in hardware without the use of processors. For example, analog circuitry can be employed. Further, the components of the IT infrastructure **10** include communication units **94, 96, 98, 100** providing the processors **28, 80, 82, 84** an interface from which to communicate over the communication network **20**. Even more, although the foregoing components of the IT infrastructure **10** were discretely described, it is to be appreciated that the components can be combined. For example, the patient data consumers **12** and the patient data producers **18** can be the same and/or have overlap. As another example, the CDSS **16** can be integrated with the patient data consumers **18** and/or the patient data producers **12**. As yet another example, the CDSS **16**, the patient data consumers **18** and the patient data producers **12** can be combined into a standalone device independent from the communication network **20**.

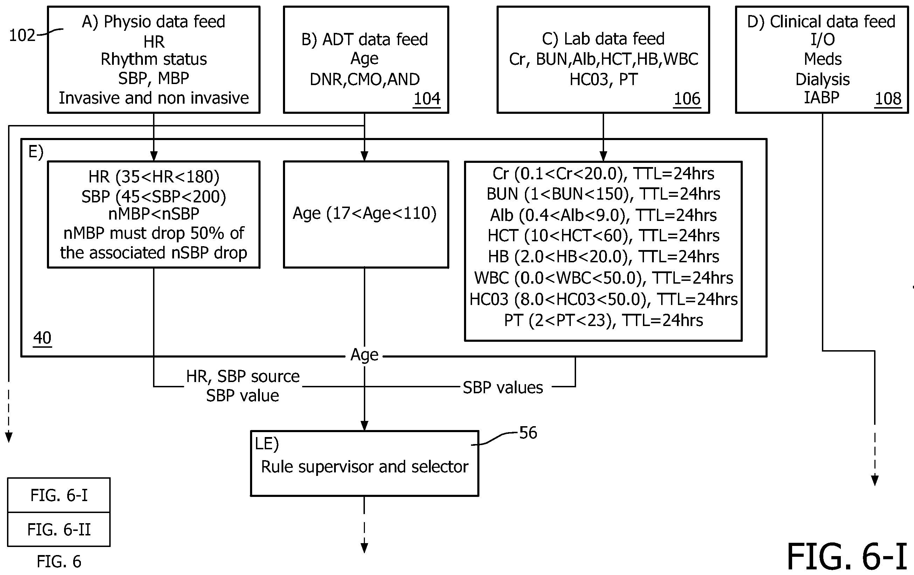

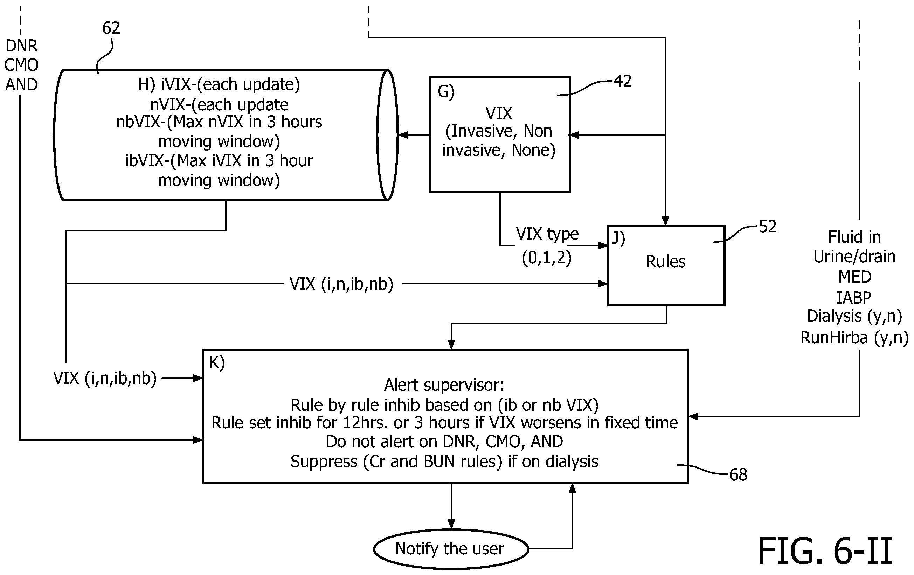

With reference to FIGURE 6, a flow chart illustrating operation of the CDSS **16** according to one embodiment thereof. The CDSS **16** receives patient data from a physiological data feed **102**, an ADT data feed **104**, a laboratory data feed **106**, and a clinical data feed **108**. As illustrated, the physiological feed **102** provides measurements for HR, SBP (invasive and/or noninvasive), mean blood pressure (MBP) (invasive and/or noninvasive), and heart rhythm status; the ADT data feed **104** provides a patient's age, whether a patient does not want to be resuscitated, whether a patient wants comfort measures only, and whether a patient wants to allow a natural death; the laboratory data feed **106** provides lab results for blood tests, including tests for creatinine (Cr), blood urea nitrogen (BUN), albumin (Alb), hematocrit (HCT), hemoglobin (HB), white blood cell (WBC) count, bicarbonate (HCO3), and prothrombin time (PT); and the clinical data feed **108** describes medications (Meds) provided to a patient, whether the patient is administered dialysis, whether a patient has a intraaortic balloon pump (IABP), any other drugs and the like administered to a patient and any samples drawn from a patient. Typically, the data feeds **102, 104, 106, 108** are the patient data producers **12**.

At least some of the patient data, including physiological data, ADT data and laboratory data, passes through the filter **40**. The filter **40** standardizes the format of the data and/or ensures the HR and SBP are within possible ranges, e.g., noninvasive MBP is less than noninvasive SBP, and the noninvasive MBP drops 50% of the associated noninvasive SBP drop. Further, the filter **40** ensures the patient age is reasonable and exceeds a predetermined age, such as the age of majority (e.g., generally 18 in the United States). Even more, the filter **40** ensures Cr, BUN, Alb, HCT, HB, WBC, HCO3 and PT are within possible ranges and the tests were performed within 24 hours (i.e., the time to live (TTL) of laboratory tests is only 24 hours).

The filtered patient data passes to the rule supervisor and selector module **56** where it is used by the rule supervisor and selector module **56** to determine a set of one or more monitoring rules and/or one or more VIX models to employ for each patient to be monitored. As illustrated, the available VIX models include a noninvasive model and an invasive model. The patient data is used to provide a context for the patient and based on this context, the appropriate monitoring rules and VIX model(s) are selected. The determination of VIX models passes to the VIX module **42**, which calculates VIX values from the filtered patient data using the selected VIX model(s). The VIX values calculated by the VIX module **42** pass to the bVIX module **62**, which calculates bVIX values from the VIX values using a three hour moving window. The determined set of monitoring rules passes to the rules module **52**, which determines thresholds for the VIX values.

The alert supervisor module **68** receives the rules and thresholds, as well as the VIX and bVIX values, and monitors for patient deterioration through application of the rule set. Upon determining deterioration, the alert supervisor **68** notifies users of patient deterioration and disarms alerts for the same deterioration. As illustrated, alerts are rearmed after 12 hours pass or 3 hours if VIX worsens. Further, the monitoring rules do not alert if the patient is DNR, CMO, or AND and/or CR and BUN rules are suppressed if the patient is on dialysis. The alert supervisor moduel **68** also has the ability to receive feedback from the user via the CDSS **16** regarding the user's desired alert behavior. In the case, the CDSS **16** is deployed where ther is no connection to interventions charted in the patient information system **14**, the user has the ability to indicate that an intervention is planned, thus supressing new alerts until the user indicates the intervention ic complete or the condition worsens to a new theshold that is based on the first alert and type of intervention indicated by the user.

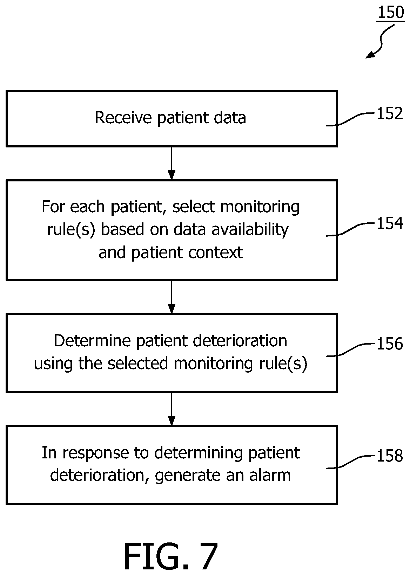

With to FIGURE 7, a block diagram of a method **150** for monitoring the patients is provided. The method **150** is suitably performed by the CDSS **16** and includes **152** patient data for the patients. The types of patient data received for the patients can vary between patients. For each patient, one or more monitoring rules are selected **154** from the rules monitoring database **60** based on patient data availability and/or patient context. As noted above, contextual data is data describing one or more of where a patient is in the care the patient's problem list, interventions, demographics, laboratory tests, and the like. Patient deterioration of the patient is determined **156** using the selected monitoring rules for the patient, and,

in response to determining patient deterioration, an alert is generated **58**. In this way, each patient is monitored with a set of rules tailored to them.

As used herein, a memory includes one or more of a non-transient computer readable medium; a magnetic disk or other magnetic storage medium; an optical disk or other optical storage medium; a random access memory (RAM), read-only memory (ROM), or other electronic memory device or chip or set of operatively interconnected chips; an Internet/Intranet server from which the stored instructions may be retrieved via the Internet/Intranet or a local area network; or so forth. Further, as used herein, a processor includes one or more of a microprocessor, a microcontroller, a graphic processing unit (GPU), an application-specific integrated circuit (ASIC), a field-programmable gate array (FPGA), and the like; a user input device includes one or more of a mouse, a keyboard, a touch screen display, one or more buttons, one or more switches, one or more toggles, and the like; a display device includes one or more of a liquid crystal display (LCD), an light emitting diode (LED) display, a plasma display, a projection display, a touch screen display, and the like; and databases include one or more memories.

The invention has been described with reference to the preferred embodiments. Modifications and alterations may occur to others upon reading and understanding the preceding detailed description. For example, while VIX was discussed as a means of detecting unstable patients, VIX could also be used to determine which patients are in a safely stable state in order to prioritize his/her time or to decide who can be moved from, for example, the ICU to the general ward, since a prolonged, very low VIX value is indicative of a very stable patient. It is intended that the invention be construed as including all such modifications and alterations insofar as they come within the scope of the appended claims.

**Claims**

1. A clinical decision
   support system **(16)** for monitoring one or more patients, said system **(16)** comprising:

   one or more processors **(84)** programmed to:

   receive patient data for the patients;
   for each patient, select one or more monitoring rules from a plurality of monitoring rules based on vital signs instability index (VIX) value of said patient;
   determine or predict whether a patient is deteriorating using the selected monitoring rules for the patient; and,
   in response to determining the patient is deteriorating, generate an alert, wherein the processors **(84)** are further programmed to:

calculate a baseline vital signs instability index (VIX) for the patient from VIX values within a past predetermined amount of time, wherein the baseline VIX indicating how the VIX has been behaving within the past predetermined amount of time;

compare the baseline VIX to the VIX threshold; and,

in response to the VIX exceeding the VIX threshold and the baseline VIX exceeding a predetermined fraction of the VIX threshold, determine that the patient is deteriorating.

2. The clinical decision support system **(16)** according to claim 1, wherein the monitoring rules for each patient are further selected based upon patient data source.

3. The clinical decision support system **(16)** according to either one of claims 1 and 2, wherein a patient's context includes one or more of where the patient is in a care process, a problem list of the patient, clinical interventions for the patient, demographics of the patient, and laboratory tests performed on the patient.

4. The clinical decision support system **(16)** according to anyone of claims 1-3, wherein the processors **(84)** are further programmed to:

   select a predictive model of stability of a physiological condition for the patient based on patient data availability and/or patient context; and, calculate a vital signs index (VIX) for the patient from the patient data using the predictive model; wherein the determining includes comparing the VIX to a VIX threshold of instability.

5. The clinical decision support system **(16)** according to any one of claims 1-4, wherein the clinical decision support system **(16)** adapts to available data and/or natively processes patient data format differently between hosts.

6. The clinical decision support system **(16)** according to claim 1, wherein the baseline VIX is a maximum VIX during the past predetermined amount of time.

7. The clinical decision support system **(16)** according to any one of claims 1-6, wherein the processors **(84)** are further programmed to:
   filter the received patient data to remove patient data failing to meet one or more of normalcy ranges, time criteria for usability, and cross parameter checks.

8. The clinical decision support system **(16)** according to anyone of claims 1-7, wherein the processors **(84)** are further programmed to:
   in response to determining the patient is deteriorating from an index or parameter of stability, disarm future alerts for the same deterioration until at least one of a plurality of rearming conditions are met, the rearming conditions including:

   a first rearming condition, which includes:

      a first predetermined amount of time passed;
      no intervention was administered during the first predetermined amount of time; and, the index or parameter worsened by a predetenrmined amount compared to the value of the index or parameter when the patient deteriorated; and,

   a second rearming condition, which includes:

      the first predetermined amount of time passed;
      intervention was administered during the first predetermined amount of time; and, a second predetermined amount of time passed.

9. The clinical decision support system **(16)** according to claim 1, wherein the patient data for the patients includes one or more of physiological data, admission, discharge and transfer (ADT) data, laboratory data, clinical data, outcome data, and/or alert data.

10. The clinical decision support system **(16)** according to claim 1, wherein the patient contextual data includes laboratory data and/or demographic data.

11. A computer-implemented method **(150)** for monitoring one or more patients, said method **(150)** comprising:

   receiving **(152)** patient data for the patients;
   for each patient, selecting **(154)** one or more monitoring rules from a plurality of monitoring rules;
   determining **(156)** whether a patient is deteriorating using the selected monitoring rules for the patient based on vital signs instability index (VIX) value of said patient; and,
   in response to determining the patient is deteriorating, generating **(158)** an alert further including:

      calculating a baseline VIX for the patient from VIX values within a past predetermined amount of time, wherein the baseline VIX indicating how the VIX has been behaving within the past predetermined amount of time;
      comparing the baseline VIX to the VIX threshold; and,

in response to the VIX exceeding the VIX threshold and the baseline VIX exceeding a predetermined fraction of the VIX threshold, determining the patient is deteriorating.

12. The computer-implemented method **(150)** according to claim 11, wherein a patient's context includes one or more of where the patient is in a care process, a problem list of the patient, clinical interventions for the patient, demographics of the patient, and laboratory tests performed on the patient.

13. The computer-implemented method **(150)** according to either one of claims 11 and 12, further including:

selecting a predictive model of stability of a physiological condition for the patient based on patient data availability and/or patient context; and, calculating a vital signs index (VIX) for the patient from the patient data using the predictive model;

wherein the determinating includes comparing the VIX to a VIX threshold of instability.

14. One or more processors **(84)** programmed to perform the method **(150)** according to anyone of claims 11-13.

**Patentansprüche**

1. Klinisches Entscheidungsunterstützungssystem (16) zum Überwachen eines oder mehrerer Patienten, wobei das System (16) Folgendes umfasst:

einen oder mehrere Prozessoren (84), die programmiert sind, um:

Patientendaten für die Patienten zu empfangen;

für jeden Patienten eine oder mehrere Überwachungsregeln aus einer Vielzahl von Überwachungsregeln basierend auf einem Wert des Vitalzeicheninstabilitätsindex (VIX) des Patienten auszuwählen; zu bestimmen oder vorherzusagen, ob sich ein Patient verschlechtert, unter Verwendung der ausgewählten Überwachungsregeln für den Patienten; und in Reaktion auf das Bestimmen, dass sich der Patient verschlechtert, eine Warnung zu generieren, wobei die Prozessoren (84) weiter programmiert sind, um:

einen Baseline-Vitalzeicheninstabilitätsindex (VIX) für den Patienten aus VIX-Werten innerhalb einer vergangenen vorgegebenen Zeitmenge zu berechnen, wobei der Baseline-VIX anzeigt, wie sich der VIX innerhalb der vergangenen vorgegebenen Zeitmenge verhalten hat; den Baseline-VIX mit dem VIX-Grenzwert zu vergleichen; und in Reaktion darauf, dass der VIX den VIX-Grenzwert überschreitet und der Baseline-VIX einen vorgegebenen Bruchteil des VIX-Grenzwerts überschreitet, zu bestimmen, dass sich der Patient verschlechtert.

2. Klinisches Entscheidungsunterstützungssystem (16) nach Anspruch 1, wobei die Überwachungsregeln für jeden Patienten weiter basierend auf einer Patientendatenquelle ausgewählt werden.

3. Klinisches Entscheidungsunterstützungssystem (16) nach einem der Ansprüche 1 und 2, wobei ein Kontext des Patienten eines oder mehrere von folgenden beinhaltet: wo sich der Patient in einem Behandlungsprozess befindet, einer Problemliste des Patienten, klinischen Interventionen für den Patienten, demografischen Daten des Patienten und bei dem Patienten durchgeführten Labortests.

4. Klinisches Entscheidungsunterstützungssystem (16) nach einem der Ansprüche 1-3, wobei die Prozessoren (84) weiter programmiert sind, um:

ein prädiktives Stabilitätsmodell einer physiologischen Bedingung für den Patienten basierend auf Patientendatenverfügbarkeit und/oder Patientenkontext auszuwählen; und einen Vitalzeichenindex (VIX) für den Patienten aus den Patientendaten unter Verwendung des prädiktiven Modells zu berechnen; wobei das Bestimmen das Vergleichen des VIX mit einem VIX-Instabilitätsgrenzwert beinhaltet.

5. Klinisches Entscheidungsunterstützungssystem (16) nach einem der Ansprüche 1-4, wobei sich das klinische Entscheidungsunterstützungssystem (16) an verfügbare Daten anpasst und/oder ein Patientendatenformat zwischen Hosts unterschiedlich nativ verarbeitet.

6. Klinisches Entscheidungsunterstützungssystem (16) nach Anspruch 1, wobei der Baseline-VIX ein maximaler VIX während der vergangenen vorgegebenen Zeitmenge ist.

7. Klinisches Entscheidungsunterstützungssystem (16) nach einem der Ansprüche 1-6, wobei die Prozessoren (84) weiter programmiert sind, um: die empfangenen Patientendaten zu filtern, um Patientendaten, die eines oder mehrere von Normalitätsbereichen, Zeitkriterien für Verwendbarkeit und Parameterabgleichen nicht erfüllen, zu entfernen.

**8.** Klinisches Entscheidungsunterstützungssystem (16) nach einem der Ansprüche 1-7, wobei die Prozessoren (84) weiter programmiert sind, um:
in Reaktion auf das Bestimmen, dass sich der Patient verschlechtert, aus einem Stabilitätsindex oder -parameter zukünftige Warnungen für die gleiche Verschlechterung zu deaktivieren, bis mindestens eine einer Vielzahl von Aktivierungsbedingungen erfüllt ist, wobei die Neuaktivierungsbedingungen Folgendes beinhalten:

eine erste Neuaktivierungsbedingung, die Folgendes beinhaltet:

eine erste vorgegebene Zeitmenge ist verstrichen;
während der ersten vorgegebenen Zeitmenge wurde keine Intervention verabreicht;
der Index oder Parameter hat sich um eine vorgegebene Menge verschlechtert im Vergleich zu dem Wert des Index oder Parameters, wenn sich der Patient verschlechtert hat; und

eine zweite Neuaktivierungsbedingung, die Folgendes beinhaltet:

die erste vorgegebene Zeitmenge ist verstrichen;
während der ersten vorgegebenen Zeitmenge wurde eine Intervention verabreicht; und
eine zweite vorgegebene Zeitmenge ist verstrichen.

**9.** Klinisches Entscheidungsunterstützungssystem (16) nach Anspruch 1, wobei die Patientendaten für die Patienten eines oder mehrere von physiologischen Daten, Aufnahme-, Entlassungs- und Überweisungsdaten (ADT-Daten), Labordaten, klinischen Daten, Ergebnisdaten und/oder Warnungsdaten beinhalten.

**10.** Klinisches Entscheidungsunterstützungssystem (16) nach Anspruch 1, wobei die Patientenkontextdaten Labordaten und/oder demografische Daten beinhalten.

**11.** Computerimplementiertes Verfahren (150) zum Überwachen eines oder mehrerer Patienten, wobei das Verfahren (150) Folgendes umfasst:

Empfangen (152) von Patientendaten für die Patienten;
Auswählen (154), für jeden Patienten, einer oder mehrerer Überwachungsregeln aus einer Vielzahl von Überwachungsregeln;

Bestimmen (156), ob sich ein Patient verschlechtert, unter Verwendung der ausgewählten Überwachungsregeln für den Patienten basierend auf einem Wert des Vitalzeicheninstabilitätsindex (VIX) des Patienten; und
Generieren (158) einer Warnung in Reaktion auf das Bestimmen, dass sich der Patient verschlechtert, weiter beinhaltend:

Berechnen eines Baseline-VIX für den Patienten aus VIX-Werten innerhalb einer vergangenen vorgegebenen Zeitmenge, wobei der Baseline-VIX anzeigt, wie sich der VIX innerhalb der vergangenen vorgegebenen Zeitmenge verhalten hat;
Vergleichen des Baseline-VIX mit dem VIX-Grenzwert; und
Bestimmen, in Reaktion darauf, dass der VIX den VIX-Grenzwert überschreitet und der Baseline-VIX einen vorgegebenen Bruchteil des VIX-Grenzwerts überschreitet, dass sich der Patient verschlechtert.

**12.** Computerimplementiertes Verfahren (150) nach Anspruch 11, wobei ein Kontext des Patienten eines oder mehrere von folgenden beinhaltet: wo sich der Patient in einem Behandlungsprozess befindet, einer Problemliste des Patienten, klinischen Interventionen für den Patienten, demografischen Daten des Patienten und bei dem Patienten durchgeführten Labortests.

**13.** Computerimplementiertes Verfahren (150) nach einem der Ansprüche 11 und 12, weiter beinhaltend:

Auswählen eines prädiktiven Stabilitätsmodells einer physiologischen Bedingung für den Patienten basierend auf Patientendatenverfügbarkeit und/oder Patientenkontext; und
Berechnen eines Vitalzeichenindex (VIX) für den Patienten aus den Patientendaten unter Verwendung des prädiktiven Modells;
wobei das Bestimmen das Vergleichen des VIX mit einem VIX-Instabilitätsgrenzwert beinhaltet.

**14.** Ein oder mehrere Prozessoren (84), die programmiert sind, um das Verfahren (150) nach einem der Ansprüche 11-13 durchzuführen.

**Revendications**

**1.** Système d'aide à la décision clinique (16) pour surveiller un ou plusieurs patients, ledit système (16) comprenant :
un ou plusieurs processeurs (84) programmés pour :

recevoir des données de patient pour les patients ;

pour chaque patient, sélectionner une ou plusieurs règles de surveillance dans une pluralité de règles de surveillance basées sur une valeur de l'indice d'instabilité des signes vitaux (VIX) dudit patient ;

déterminer ou prédire si un patient se détériore en utilisant les règles de surveillance sélectionnées pour le patient ; et

en réponse à la détermination de la détérioration du patient, générer une alerte, dans lequel les processeurs (84) sont en outre programmés pour :

calculer un indice d'instabilité des signes vitaux (VIX) de référence pour le patient à partir des valeurs VIX dans un laps de temps prédéterminé au préalable, dans lequel le VIX de référence indique comment le VIX s'est comporté au cours du laps de temps prédéterminé au préalable ;

comparer le VIX de référence à la valeur seuil de VIX ; et

en réponse au VIX dépassant la valeur seuil de VIX et au VIX de référence dépassant une fraction prédéterminée de la valeur seuil de VIX, déterminer que le patient se détériore.

2. Système d'aide à la décision clinique (16) selon la revendication 1, dans lequel les règles de surveillance pour chaque patient sont en outre sélectionnées sur la base d'une source de données du patient.

3. Système d'aide à la décision clinique (16) selon l'une ou l'autre des revendications 1 et 2, dans lequel le contexte du patient inclut un ou plusieurs parmi le niveau où en est le patient dans un processus de soin, une liste de problèmes du patient, des interventions cliniques du patient, des données démographiques du patient et des analyses de laboratoire réalisées sur le patient.

4. Système d'aide à la décision clinique (16) selon l'une quelconque des revendications 1 à 3, dans lequel les processeurs (84) sont en outre programmés pour :

sélectionner un modèle prédictif de stabilité d'un état physiologique pour le patient sur la base de la disponibilité des données du patient et/ou du contexte du patient ; et

calculer un indice de signes vitaux (VIX) pour le patient à partir des données du patient en utilisant le modèle prédictif ;

dans lequel la détermination inclut une comparaison du VIX à une valeur seuil d'instabilité de VIX.

5. Système d'aide à la décision clinique (16) selon l'une quelconque des revendications 1 à 4, dans lequel le système d'aide à la décision clinique (16) s'adapte aux données disponibles et/ou traite de manière native un format de données de patient différemment entre les hôtes.

6. Système d'aide à la décision clinique (16) selon la revendication 1, dans lequel le VIX de référence est un VIX maximum pendant le laps de temps prédéterminé au préalable.

7. Système d'aide à la décision clinique (16) selon l'une quelconque des revendications 1 à 6, dans lequel les processeurs (84) sont en outre programmés pour :

filtrer les données du patient reçues pour éliminer des données du patient ne répondant pas à un ou plusieurs parmi des plages de normalité, des critères temporels d'utilité et des contrôles de paramètres croisés.

8. Système d'aide à la décision clinique (16) selon l'une quelconque des revendications 1 à 7, dans lequel les processeurs (84) sont en outre programmés pour :

en réponse à la détermination de la détérioration du patient à partir d'un indice ou d'un paramètre de stabilité, désamorcer des alertes futures pour la même détérioration jusqu'à ce qu'au moins une pluralité de conditions de réamorçage soient satisfaites, les conditions de réamorçage comprenant :

une première condition de réamorçage, qui inclut :

l'écoulement d'un premier laps de temps prédéterminé ;

une absence d'intervention exécutée pendant le premier laps de temps déterminé ; et,

une aggravation de l'indice ou du paramètre d'un niveau prédéterminé par rapport à la valeur de l'indice ou paramètre lorsque le patient se détériore ; et

une seconde condition de réamorçage, qui inclut :

l'écoulement du premier laps de temps prédéterminé ;

l'exécution d'une intervention pendant le premier laps de temps déterminé ; et

l'écoulement d'un second laps de temps.

9. Système d'aide à la décision clinique (16) selon la revendication 1, dans lequel les données du patient pour les patients incluent une ou plusieurs parmi des données physiologiques, des données d'hospitalisation, de sortie d'hôpital et de transfert (ADT), des données de laboratoire, des données cliniques, des données d'évolution, et/ou des données d'alerte.

10. Système d'aide à la décision clinique (16) selon la revendication 1, dans lequel les données contextuelles du patient incluent des données de laboratoire et/ou des données démographiques.

11. Procédé mis en oeuvre par ordinateur (150) pour surveiller un ou plusieurs patients, ledit procédé (150) comprenant :

la réception (152) de données de patient pour les patients ;
pour chaque patient, la sélection (154) d'une ou plusieurs règles de surveillance parmi une pluralité de règles de surveillance ;
la détermination (156) de la détérioration éventuelle d'un patient en utilisant les règles de surveillance sélectionnées pour le patient sur la base d'une valeur de l'indice d'instabilité des signes vitaux (VIX) dudit patient ; et
en réponse à la détermination de la détérioration du patient, la génération (158) d'une alerte, incluant en outre :

le calcul d'un VIX de référence pour le patient à partir des valeurs VIX dans un laps de temps prédéterminé au préalable, dans lequel le VIX de référence indique comment le VIX s'est comporté au cours du laps de temps prédéterminé au préalable ;
la comparaison du VIX de référence à la valeur seuil de VIX ; et
en réponse à un VIX dépassant la valeur seuil de VIX et au VIX de référence dépassant une fraction prédéterminée de la valeur seuil de VIX, la détermination de la détérioration du patient.

12. Procédé mis en oeuvre par ordinateur (150) selon la revendication 11, dans lequel le contexte du patient inclut un ou plusieurs parmi le niveau où en est le patient dans un processus de soin, une liste de problèmes du patient, des interventions cliniques du patient, des données démographiques du patients et des analyses de laboratoire réalisées sur le patient.

13. Procédé mis en oeuvre par ordinateur (150) selon l'une ou l'autre des revendications 11 et 12, incluant en outre :

la sélection d'un modèle prédictif de stabilité d'un état physiologique pour le patient sur la base de la disponibilité de données du patient et/ou du contexte du patient ; et
le calcul d'un indice de signes vitaux (VIX) pour le patient à partir des données du patient en utilisant le modèle prédictif ;
dans lequel la détermination inclut une comparaison du VIX à une valeur seuil d'instabilité de VIX.

14. Un ou plusieurs processeurs (84) programmés pour réaliser le procédé (150) selon l'une quelconque des revendications 11 à 13.

FIG. 1

EP 2 795 495 B1

FIG. 2

FIG. 3

FIG. 4

FIG. 5

102 —

| A) Physio data feed<br>HR<br>Rhythm status<br>SBP, MBP<br>Invasive and non invasive | B) ADT data feed<br>Age<br>DNR,CMO,AND<br><u>104</u> | C) Lab data feed<br>Cr, BUN,Alb,HCT,HB,WBC<br>HC03, PT<br><u>106</u> | D) Clinical data feed<br>I/O<br>Meds<br>Dialysis<br>IABP   <u>108</u> |

E)

| HR (35<HR<180)<br>SBP (45<SBP<200)<br>nMBP<nSBP<br>nMBP must drop 50% of<br>the associated nSBP drop<br><br><u>40</u> | Age (17<Age<110) | Cr (0.1<Cr<20.0), TTL=24hrs<br>BUN (1<BUN<150), TTL=24hrs<br>Alb (0.4<Alb<9.0), TTL=24hrs<br>HCT (10<HCT<60), TTL=24hrs<br>HB (2.0<HB<20.0), TTL=24hrs<br>WBC (0.0<WBC<50.0), TTL=24hrs<br>HC03 (8.0<HC03<50.0), TTL=24hrs<br>PT (2<PT<23), TTL=24hrs |

———————————— Age ————————————

HR, SBP source
SBP value ———————————— SBP values ————

—56

LE)
Rule supervisor and selector

| FIG. 6-I |
|---|
| FIG. 6-II |

FIG. 6

# FIG. 6-I

62

DNR
CMO
AND

H) iVIX-(each update)
nVIX-(each update
nbVIX-(Max nVIX in 3 hours
moving window)
ibVIX-(Max iVIX in 3 hour
moving window)

G)
VIX
(Invasive, Non
invasive, None)

42

VIX type
(0,1,2)

J)
Rules

52

VIX (i,n,ib,nb)

Fluid in
Urine/drain
MED
IABP
Dialysis (y,n)
RunHirba (y,n)

VIX (i,n,ib,nb)

K)
Alert supervisor:
Rule by rule inhib based on (ib or nb VIX)
Rule set inhib for 12hrs. or 3 hours if VIX worsens in fixed time
Do not alert on DNR, CMO, AND
Suppress (Cr and BUN rules) if on dialysis

68

Notify the user

FIG. 6-II

_150_

| Receive patient data | ⎯152 |

↓

| For each patient, select monitoring rule(s) based on data availability and patient context | ⎯154 |

↓

| Determine patient deterioration using the selected monitoring rule(s) | ⎯156 |

↓

| In response to determining patient deterioration, generate an alarm | ⎯158 |

# FIG. 7

**EP 2 795 495 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006136972 A